# EUROPEAN PATENT SPECIFICATION

(11) **EP 2 234 998 B1**
(45) Date of publication and mention of the grant of the patent: **19.08.2015**
(21) Application number: 08863604.8
(22) Date of filing: 19.12.2008
(51) Int. Cl.: C07D 401/10, C07D 475/08, A61K 31/505

(54) **SPECIFIC INHIBITORS OF PTERIDINE REDUCTASE WITH ANTIPARASITIC ACTION**
SPEZIFISCHE INHIBITOREN VON PTERIDINREDUKTASE MIT ANTIPARASITÄRER WIRKUNG
INHIBITEURS SPÉCIFIQUES DE LA PTÉRIDINE RÉDUCTASE À ACTION ANTIPARASITAIRE

(30) Priority: 24.12.2007 IT MI20072426
(43) Date of publication of application: 06.10.2010
(73) Proprietor: Tydock Pharma S.r.l., 41126 Modena (IT)
(72) Inventor: COSTI, Maria Paola, 41100 Modena (IT); PAGLIETTI, Giuseppe, 07100 Sassari (IT)
(74) Representative: Biggi, Cristina
(86) International application number: PCT/EP2008/011106
(87) International publication number: WO 2009/080367

(56) References cited:
- CHAYKOVSKY M ET AL: "METHOTREXATE ANALOGS. 6. REPLACEMENT OF GLUTAMIC ACID BY VARIOUS AMINO ACID ESTERS AND AMINES" JOURNAL OF MEDICINAL CHEMISTRY, AMERICAN CHEMICAL SOCIETY, WASHINGTON, US, vol. 18, no. 8, 1 January 1975 (1975-01-01), pages 909-912, XP000906980 ISSN: 0022-2623
- DAWSON ALICE ET AL: "Structure and reactivity of Trypanosoma brucei pteridine reductase: inhibition by the archetypal antifolate methotrexate." MOLECULAR MICROBIOLOGY SEP 2006, vol. 61, no. 6, September 2006 (2006-09), pages 1457-1468, XP002532613 ISSN: 0950-382X
- HARDY L W ET AL: "Biochemical and genetic tests for inhibitors of Leishmania pteridine pathways." EXPERIMENTAL PARASITOLOGY NOV 1997, vol. 87, no. 3, November 1997 (1997-11), pages 157-169, XP002532614 ISSN: 0014-4894
- CAVAZZUTI ANTONIO ET AL: "Discovery of potent pteridine reductase inhibitors to guide antiparasite drug development." PROCEEDINGS OF THE NATIONAL ACADEMY OF SCIENCES OF THE UNITED STATES OF AMERICA 5 FEB 2008, vol. 105, no. 5, 5 February 2008 (2008-02-05), pages 1448-1453, XP002532615 ISSN: 1091-6490

## Description

The present invention relates to 4-(heteroaryl)aminobenzoyl derivative compounds, to processes for their preparation, to pharmaceutical compositions containing them, and to the use of said compounds as synergistic agents in the manufacture of pharmaceutical composition for the combination treatment of protozoarian infections, such as, for example, Leishmaniasis and Trypanosomiasis. Protozoan parasites of the order Kinetoplastida are the causal agents of serious human diseases including African sleeping sickness, Chagas' disease and Leishmaniasis.

Therefore, there is an urgent need for new more effective drugs targeting these neglected diseases since those in current use are toxic, expensive and often difficult to administer.

The problem is compounded by an increase in drug resistance and lack of progress in drug development. Only a single neweffective treatment has been developed in the last 25 years, Miltefosine (hexadecylphosphocholine).

Enzymes involved in the provision and use of reduced folate cofactors such as dihydrofolate reductase (DHFR) and thymidylate synthase (TS) are valued drug targets for the treatment of certain parasitic diseases.

DHFR catalyses the two-step reduction of folate to tetrahydrofolate, which is then transformed to N⁵, N¹⁰-methylene tetrahydrofolate and is used by TS as a methyldonor and reducing agent in the conversion of 2'-deoxy-uridine-5'-monophosphate to 2'-deoxy-thymidine-5'-monophosphate.

Inhibition of DHFR or TS reduces the cellular pool of 2'-deoxy-thymidine-5'-monophosphate, impairing DNA replication and resulting in cell death.

Since trypanosomatids are auxotrophic for folate and pterins, the inhibition of the enzymes dependent on them should provide suitable treatments.

However, Leishmania and Trypanosoma parasites are not sensitive to classical antifolates that are normally effective against malaria and other parasitic diseases.

It is known that this is due to the presence of a specific enzyme, pteridine reductase 1 (PTR1), that functions as bypass in case of dihydrofolate reductase inhibition. Therefore, antifolates are not employed in the therapy of trypanosomatid infections mainly because of pteridine reductase (PTR1).

While the bifunctional DHFR-TS used by trypanosomatids can exclusively reduce folic acid, the short-chain dehydrogenase/reductase PTR1 shows a much broader range of activity catalysing successive reductions of conjugated (folate) and unconjugated (biopterin) pterins.

Under physiological conditions PTR1 is responsible of reduction of about 10% of the folic acid required by the cell, but when classical antifolate drugs inhibit DHFR-TS, PTR1 can be overexpressed, allowing for significant reduction of the necessary amounts of folates to ensure parasite survival.

This compensatory mechanism suggests that treatment of trypanosomatid infections could be achieved through inhibition of both DHFR and PTR1 by a single drug or a combination of drugs that are specific inhibitors of the two targets.

When the compounds of the present invention were tested against the intracellular form of the parasite, the amastigote form for Leishmania major and Trypanosoma cruzi cell lines, in combination with different well known antifolate compounds such as methotrexate, pyrimethamine (PYR), aminopterine, trimetoprim, surprisingly and unexpectedly they showed a synergistic/additive antiparasitic profile, which paralleled the profile observed in the cell lines of the promastigote forms. Compounds derived from folate analogs as inhibitors of Pteridine reductase for antiparasitic use are described, for example, in:
- Sundar S, Makharia A, More D-K, Agrawal G, Voss A, Fischer C, Bachmann P, Murray H-W (2000) Clin Infect Dis 31:1110-1113.
- Then R-L (2004) J Chemother 16:3-12.
- McGuire J-J (2003) Curr Pharm Des 9:2593-2613.
- Gregson A, Plowe C-V (2005) Pharmacol Rev 57:117-145.
- Senkovich O, Pal B, Schormann N, Chattopadhyay D (2003) Mol Biochem Parasitol 127:89-92.
- Cunningham M-L, Titus R-G, Turco S-J, Beverley S-M (2001) Science 292:285-287.
- Nare B, Luba J, Hardy L-W, Beverley S (1997) Parasitology 114 Suppl:S101-110.
- Bello A-R, Nare B, Freedman D, Hardy L, Beverley S-M (1994) Proc Natl Acad Sci USA 91:11442-11446.
- Hardy L-W, Matthews W, Nare B, Beverley S-M (1997) Exp Parasitol 87:157-169.
- Gourley D-G, Schüttelkopf A-W, Leonard G-A, Luba J, Hardy L-W, Beverley S-M, Hunter WN (2001) Nat Struct Biol 8:521-525.
- Schuttelkopf A-W, Hardy L-W, Beverley S-M, Hunter W-N (2005) J MoI Biol 352(1):105-116.
- Dawson A, Gibellini F, Sienkiewicz N, Tulloch L-B, Fyfe P-K, McLuskey K, Fairlamb A-H, Hunter W-N (2006). Mol Microbiol 61(6): 1457-1468

The inventors believe that the compounds of the present invention exert their activity in virtue of their ability to specifically inhibit both enzymes pteridine reductase 1 and dihydrofolate reductase at the same time alone or in combination with classical antifolates.

Compounds that are the object of the invention are included in the general formula (I): 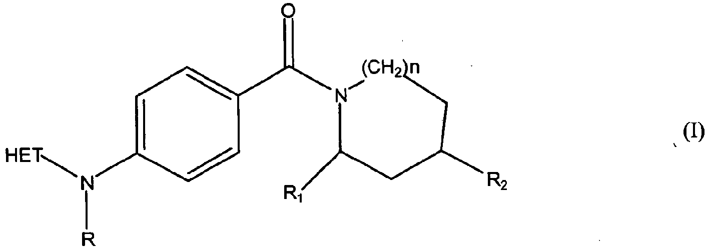 wherein:
R is hydrogen or a branched or straight chain C1-C6 alkyl; optionally non substituted or substituted with hydroxyl, halogen, carboxyl, alkoxy, carbonyl;
R₁ and R₂ are each independently hydrogen or COORₐ, wherein R₃ is hydrogen or a branched or straight chain C1-C6 alkyl; n is an integer which may be 0, or 1.
HET is a 2,4-diaminopteridin-6-ylmethyl moiety of the following formula (II): 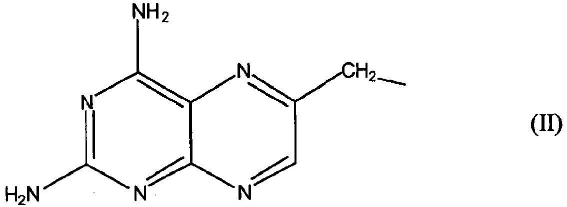 or a quinoxalin-2-yl moiety represented by the following formula (III): 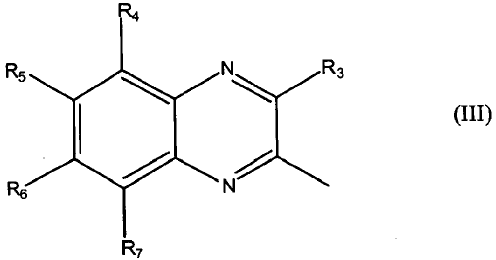 wherein:
   R₃ is hydrogen, branched or straight chain C1-C6 alkyl, unsubstituted phenyl or COORₐ, wherein R₃ is hydrogen or a branched or straight chain C1-C6 alkyl;
   R₄ and R₇ are each independently hydrogen or NR_{b}R_{c}, wherein
   R₃ and R_{b}, which may be the same or different, are hydrogen or a branched or straight chain C1-C6 alkyl;
   R₅ and R₆ are each independently hydrogen or CF₃.

The object of the present invention is a compound having the following general formula (Ia): 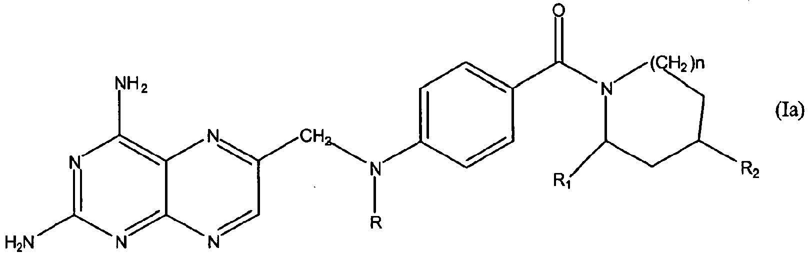 wherein:
R is hydrogen or CH₃, CH2CH2OH, COOH, COOCH3, COOC2H5;
R1 is hydrogen or COOH; R2 is hydrogen, COOH or COOCH3; and;
and n is 0 or 1.

Compounds having the following general formula (Ib) are not the object of the claims: 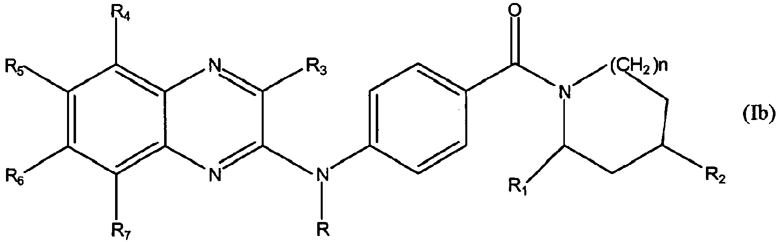 wherein:
R is hydrogen or CH3;
R₁ and R₂ are each independently hydrogen, COOH, or COOCH₃;
R₃ is hydrogen, CH₃, C₆H₅, COOH, or COOC₂H₅;
R₄ and R₇ are both hydrogen;
R₅ is CF₃ and R₆ is hydrogen;
and n is 0 or 1.

Examples of preferred specific compounds under this invention are the compounds of formula (Ia):
1-[4-(2,4-diaminopteridin-6-ylmethylamino)benzoyl]piperidin-2-carboxylic acid.
1-[4-(2,4-diaminopteridin-6-ylmethylamino)benzoyl]piperidin-4-carboxylic acid.
1-[4-(2,4-diaminopteridin-6-ylmethylamino)benzoyl]piperidin-2,4-dicarboxylic acid.
1-[4-(2,4-diaminopteridin-6-ylmethylamino)benzoyl]pyrrolidin-2-carboxylic acid.
1-[4-(2,4-diaminopteridin-6-ylmethylamino)benzoyl]pyrrolidin-4-carboxylic acid.
1-[4-(2,4-diaminopteridin-6-ylmethylamino)benzoyl]pyrrolidin-2,4-dicarboxylic acid.
1-{4-[(2,4-diaminopteridin-6-ylmethyl)(methyl)amino]benzoyl}pyrrolidin-2-carboxylic acid.
1-{4-[(2,4-diaminopteridin-6-ylmethyl)(methyl)amino]benzoyl}pyrrolidin-4-carboxylic acid.
1-{4-[(2,4-diaminopteridin-6-ylmethyl)(methyl)amino]benzoyl}pyrrolidin-2,4-dicarboxylic acid.
1-{4-[(2-4-diaminopteridin-6-ylmethyl)(methyl)amino]benzoyl}piperidin-2-carboxylic acid.
1-{4-[(2,4-diaminopteridin-6-ylmethyl)(methyl)amino]benzoyl}piperidin-4-carboxylic acid and
1-{4-[(2,4-diaminopteridin-6-ylmethyl)(methyl)amino]benzoyl}piperidin-2,4-dicarboxylic acid
1-[4 -(2, 4-diaminopteridin-6-ylmethylamino)benzoyl]-piperidin-4-carboxyl-methyl ester methyl 1-{4-[(2,4-diaminopteridin-6-ylmethyl) (methyl)amino]benzoyl}piperidine-4-carboxylate
1-{4-(2,4-diaminopteridin-6-ylmethyl)(2-ethoxy-2-oxoethyl)amino]benzoyl} 4-carboxymethyl piperidine which does not fall within the scope of formula (Ia)
1-{[4-(2,4-diaminopteridin-6-ylmethyl)(2-hydroxyethyl)amino]benzoyl}4-carboxymethyl piperidine
1-(4-((2,4-diaminopteridin-6-ylmethylamino) benzoyl) piperidine 4-carboxyamide which does not fall within the scope of formula (Ia)

Examples of compounds of formula (Ib) which are not part of the invention are:
1-[4-(6-trifluoromethylquinoxalin-2-ylamino)benzoyl]pyrrolidin-2-carboxylic acid.
1-[4-(6-trifluoromethylquinoxalin-2-ylamino)benzoyl]pyrrolidin-4-carboxylic acid.
1-[4-(6-trifluoromethylquinoxalin-2-ylamino)benzoyl]pyrrolidin-2,4-dicarboxylic acid.
1-[4-(3-methyl-6-trifluoromethylquinoxalin-2-ylamino)benzoyl]pyrrolidin-2-carboxylic acid
1-[4-(3-methyl-6-trifluoromethylquinoxalin-2-ylamino)benzoyl]pyrrolidin-4-carboxylic acid
1-[4-(3-methyl-6-trifluoromethylquinoxalin-2-ylamino)benzoyl]pyrrolidin-2,4-dicarboxylic acid
1-[4-(3-phenyl-6-trifluoromethylquinoxalin-2-ylamino)benzoyl]pyrrolidin-2-carboxylic acid
1-[4-(3-phenyl-6-trifluoromethylquinoxalin-2-ylamino)benzoyl]pyrrolidin-4-carboxylic acid
1-[4-(3-phenyl-6-trifluoromethylquinoxalin-2-ylamino)benzoyl]pyrrolidin-2,4-dicarboxylic acid
1-[4-(3-methoxycarbonyl-6-trifluoromethylquinoxalin-2-ylamino)benzoyl]pyrrolidin-2-carboxylic acid
1-[4-(3-methoxycarbonyl-6-trifluoromethylquinoxalin-2-ylamino)benzoyl]pyrrolidin-4-carboxylic acid
1-[4-(3-methoxycarbonyl-6-trifluoromethylquinoxalin-2-ylamino)benzoyl]pyrrolidin-2,4-dicarboxylic acid
1-[4-(7-trifluoromethylquinoxalin-2-ylamino)benzoyl]pyrrolidin-2-carboxylic acid
1-[4-(7-trifluoromethylquinoxalin-2-ylamino)benzoyl]pyrrolidin-4-carboxylic acid
1-[4-(7-trifluoromethylquinoxalin-2-ylamino)benzoyl]pyrrolidin-2,4-dicarboxylic acid
1-[4-(3-methyl-7-trifluoromethylquinoxalin-2-ylamino)benzoyl]pyrrolidin-2-carboxylic acid
1-[4-(3-methyl-7-trifluoromethylquinoxalin-2-ylamino)benzoyl]pyrrolidin-4-carboxylic acid
1-[4-(3-methyl-7-trifluoromethylquinoxalin-2-ylamino)benzoyl]pyrrolidin-2,4-dicarboxylic acid
1-[4-(3-phenyl-7-trifluoromethylquinoxalin-2-ylamino)benzoyl]pyrrolidin-2-carboxylic acid
1-[4-(3-phenyl-7-trifluoromethylquinoxalin-2-ylamino)benzoyl]pyrrolidin-4-carboxylic acid
1-[4-(3-phenyl-7-trifluoromethylquinoxalin-2-ylamino)benzoyl]pyrrolidin-2,4-dicarboxylic acid
1-[4-(3-methoxycarbonyl-7-trifluoromethylquinoxalin-2-ylamino)benzoyl]pyrrolidin-2-carboxylic acid
1-[4-(3-methoxycarbonyl-7-trifluoromethylquinoxalin-2-ylamino)benzoyl]pyrrolidin-4-carboxylic acid
1-[4-(3-methoxycarbonyl-7-trifluoromethylquinoxalin-2-ylamino)benzoyl]pyrrolidin-2,4-dicarboxylic acid
1-[4-(6-trifluoromethylquinoxalin-2-ylamino)benzoyl]piperidin-2-carboxylic acid.
1-[4-(6-trifluoromethylquinoxalin-2-ylamino)benzoyl]piperidin-4-carboxylic acid
1-[4-(6-trifluoromethylquinoxalin-2-ylamino)benzoyl]piperidin-2,4-dicarboxylic acid
1-[4-(6-trifluoromethylquinoxalin-2-ylamino)benzoyl]piperidin-2-carboxylic acid
1-[4-(3-methyl-6-trifluoromethylquinoxalin-2-ylamino)benzoyl]piperidin-4-carboxylic acid
1-[4-(3-methyl-6-trifluoromethylquinoxalin-2-ylamino)benzoyl]piperidin-2,4-dicarboxylic acid
1-[4-(3-phenyl-6-trifluoromethylquinoxalin-2-ylamino)benzoyl]piperidin-2-carboxylic acid
1-[4-(3-phenyl-6-trifluoromethylquinoxalin-2-ylamino)benzoyl]piperidin-4-carboxylic acid
1-[4-(3-phenyl-6-trifluoromethylquinoxalin-2-ylamino)benzoyl]piperidin-2,4-dicarboxylic acid
1-[4-(3-methoxycarbonyl-6-trifluoromethylquinoxalin-2-ylamino)benzoyl]piperidin-2,4-dicarboxylic acid
1-[4-(3-methoxycarbonyl-6-trifluoromethylquinoxalin-2-ylamino)benzoyl]piperidin-4-carboxylic acid
1-[4-(3-methoxycarbonyl-6-trifluoromethylquinoxalin-2-ylamino)benzoyl]piperidin-2,4-dicarboxylic acid
1-[4-(7-trifluoromethylquinoxalin-2-ylamino)benzoyl]piperidin-2-carboxylic acid
1-[4-(7-trifluoromethylquinoxalin-2-ylamino)benzoyl]piperidin-4-carboxylic acid
1-[4-(7-trifluoromethylquinoxalin-2-ylamino)benzoyl]piperidin-2,4-dicarboxylic acid
1-[4-(3-methyl-7-trifluoromethylquinoxalin-2-ylamino)benzoyl]piperidin-2-carboxylic acid
1-[4-(3-methyl-7-trifluoromethylquinoxalin-2-ylamino)benzoyl]piperidin-4-carboxylic acid
1-[4-(3-methyl-7-trifluoromethylquinoxalin-2-ylamino)benzoyl]piperidin-2,4-dicarboxylic acid
1-[4-(3-phenyl-7-trifluoromethylquinoxalin-2-ylamino)benzoyl]piperidin-2-carboxylic acid
1-[4-(3-phenyl-7-trifluoromethylquinoxalin-2-ylamino)benzoyl]piperidin-4-carboxylic acid
1-[4-(3-phenyl-7-trifluoromethylquinoxalin-2-ylamino)benzoyl]piperidin-2,4-dicarboxylic acid
1-[4-(3-methoxycarbonyl-7-trifluoromethylquinoxalin-2-ylamino)benzoyl]piperidin-2-carboxylic acid
1-[4-(3-methoxycarbonyl-7-trifluoromethylquinoxalin-2-ylamino)benzoyl]piperidin-4-carboxylic acid
1-[4-(3-methoxycarbonyl-7-trifluoromethylquinoxalin-2-ylamino)benzoyl]piperidin-2,4-dicarboxylic acid
1-[4-(6-trifluoromethylquinoxalin-2-ylamino)benzoyl]pyrrolidin-2-carboxylic acid
1-[4-(6-trifluoromethylquinoxalin-2-ylamino)benzoyl]pyrrolidin-4-carboxylic acid
1-[4-(6-trifluoromethylquinoxalin-2-ylamino)benzoyl]pyrrolidin-4-carboxylic acid
1-[4-(3-methyl-6-trifluoromethylquinoxalin-2-ylamino)benzoyl]pyrrolidin-2-carboxylic acid
1-[4-(3-methyl-6-trifluoromethylquinoxalin-2-ylamino)benzoyl]pyrrolidin-4-carboxylic acid
1-[4-(3-methyl-6-trifluoromethylquinoxalin-2-ylamino)benzoyl]pyrrolidin-2,4-dicarboxylic acid
1-[4-(3-phenyl-6-trifluoromethylquinoxalin-2-ylamino)benzoyl]pyrrolidin-2-carboxylic acid
1-[4-(3-phenyl-6-trifluoromethylquinoxalin-2-ylamino)benzoyl]pyrrolidin-4-carboxylic acid
1-[4-(3-phenyl-6-trifluoromethylquinoxalin-2-ylamino)benzoyl]pyrrolidin-2,4-dicarboxylic acid
1-[4-(3-carboxy-6-trifluoromethylquinoxalin-2-ylamino)benzoyl]pyrrolidin-2-carboxylic acid
1-[4-(3-carboxy-6-trifluoromethylquinoxalin-2-ylamino)benzoyl]pyrrolidin-4-carboxylic acid
1-[4-(3-carboxy-6-trifluoromethylquinoxalin-2-ylamino)benzoyl]pyrrolidin-2,4-dicarboxylic acid
1-[4-(7-trifluoromethylquinoxalin-2-ylamino)benzoyl]pyrrolidin-2-carboxylic acid
1-[4-(7-trifluoromethylquinoxalin-2-ylamino)benzoyl]pyrrolidin-4-carboxylic acid
1-[4-(7-trifluoromethylquinoxalin-2-ylamino)benzoyl]pyrrolidin-2,4-dicarboxylic acid
1-[4-(3-methyl-7-trifluoromethylquinoxalin-2-ylamino)benzoyl]pyrrolidin-2-carboxylic acid
1-[4-(3-methyl-7-trifluoromethylquinoxalin-2-ylamino)benzoyl]pyrrolidin-4-carboxylic acid
1-[4-(3-methyl-7-trifluoromethylquinoxalin-2-ylamino)benzoyl]pyrrolidin-2,4-dicarboxylic acid
1-[4-(3-phenyl-7-trifluoromethylquinoxalin-2-ylamino)benzoyl]pyrrolidin-2-carboxylic acid
1-[4-(3-phenyl-7-trifluoromethylquinoxalin-2-ylamino)benzoyl]pyrrolidin-4-carboxylic acid
1-[4-(3-phenyl-7-trifluoromethylquinoxalin-2-ylamino)benzoyl]pyrrolidin-2,4-dicarboxylic acid
1-[4-(3-carboxy-7-trifluoromethylquinoxalin-2-ylamino)benzoyl]pyrrolidin-2-carboxylic acid
1-[4-(3-carboxy-7-trifluoromethylquinoxalin-2-ylamino)benzoyl]pyrrolidin-4-carboxylic acid
1-[4-(3-carboxy-7-trifluoromethylquinoxalin-2-ylamino)benzoyl]pyrrolidin-2,4-dicarboxylic acid
1-[4-(3-phenyl-7-trifluoromethylquinoxalin-2-ylamino)(methyl)benzoyl]pyrrolidin-2,4-dicarboxylic acid
1-[4-(3-carboxy-7-trifluoromethylquinoxalin-2-ylamino)(methyl)benzoyl]pyrrolidin-2-carboxylic acid
1-[4-(3-carboxy-7-trifluoromethylquinoxalin-2-ylamino)(methyl)benzoyl]pyrrolidin-4-carboxylic acid
1-[4-(3-carboxy-7-trifluoromethylquinoxalin-2-ylamino)(methyl)benzoyl]pyrrolidin-2,4-dicarboxylic acid
   - Piper, J.R.; Montgomery, J.A. Preparation of 6-(Bromomethyl)-2,4-pteridinediammine Hydrobromide and Its Use in Improved Syntheses of Methotrexate and Related Compounds. J. Org. Chem. 1977, 42, 208-211
   - Schneider, M.; Harris, T.M. Synthesis of DL-Slaframine. J. Org. Chem. 1984, 49, 3681-3684
   - Reimschneider, R.; Kassahn, H.G. Zur Herstellung von o-Diacetyl-benzol und 4-Nitro-1.2-diacetyl-benzol. Chemische Berichte. 1959, 1705-1079
   - Buhler, S.; Lagoja, I.; Giergrich, H.; Stengele, K.P.; Pfleiderer, W. New Types of Very Photolabile Protecting Groups Based upon the [2-(2-Nitrophenyl)propoxy]carbonyl (NPPOC) Moiety. Helvetica Chimica Acta 2004, 87, 620-659
   - Wieland, H.; Corner, L. Synthese des 7,11-Diathyl-pyrrochinolins (Isovomipyrin). Liebigs Ann. Chem. 1938, 536, 89
   - Piper J-R, Montgomery J-A (1974) J Het Chem 11:279-280.
   - Piper J-R, Montgomery J-A (1977) J Org Chem 42:208-211.
   - Loriga M, Piras S, Paglietti G, Costi M-P, Venturelli A (2003) Farmaco 58:51-61.

In a second aspect, the present invention provides processes for the preparation of compounds of formula (I). Specifically, the compounds of formula (Ia) according to the present invention, may be prepared by reacting 2,4-diaminopteridin-6-ylmethyl bromide hydrobromide salt of formula (IV) with an amino compound of formula (V), wherein R, R₁, R₂ and n have the meanings given above, with the proviso that R₃ is not hydrogen:

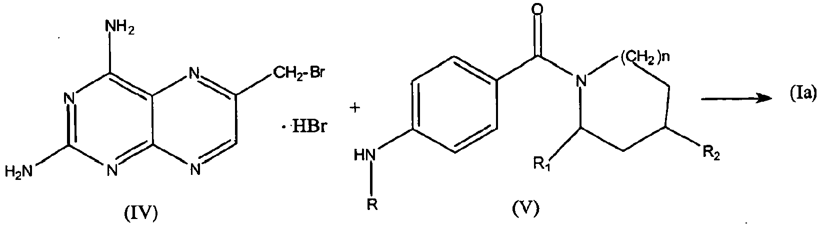

The reaction is carried out in a suitable inert solvent, such as dimethylformamide, dimethylacetamide, preferably in dimethylacetamide and at room temperature.

Intermediate V was prepared starting from the 4-nitro benzoyl derivatives that were reacted in ethanol and 10% palladised charcoal was hydrogenated and then the corresponding amine derivatives were obtained.

Specifically, the compounds of formula (Ib) which are not part of the present invention, may be prepared by reacting a 2-chloroquinoxaline of formula (VI), wherein R₃, R₄, R₅, R₆, and R₇ have the meaning given above, with an amino compound of formula (V), wherein R, R₁ R₂ and n have the meanings given above, with the proviso that R₃ is not hydrogen:

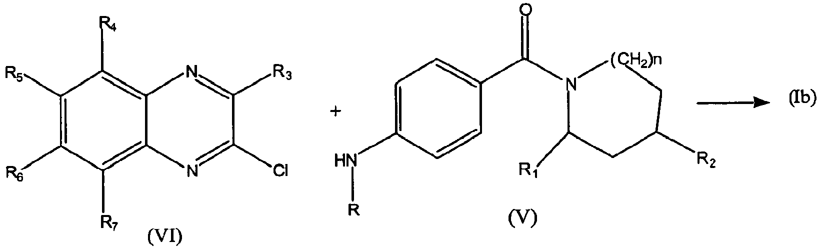

The reaction is carried out in a suitable inert solvent, preferably in an alcoholic solvent, more preferably ethanol or propanol, under reflux for a period of time ranging from 10 to 200 hours. Compounds of formula VI was prepared according to literature (3-5 ptr1 SI).

In the case when R₃ is hydrogen, the compounds of general formula (I) may be obtained by hydrolysing the corresponding compounds of formula (I) wherein R₃ is not hydrogen, following conventional hydrolytic conditions well known to an expert in the art.

According to the above processes, a compound of formula (V), wherein R is hydrogen and R₁ R₂ and n have the meaning given above, may be obtained by reducing a nitrocompound of formula (IX) following the non conventional procedure that follows:
The 4-nitro benzoyl derivatives of formula IX were obtained by reacting the commercially available or obtained with known methods (2 ref) compound of formula VIII in dimethyilformamide with compound of formula VII, p-nitrobenzoylchloride. The reactions were conducted preferably at room temperature in nitrogen atmosphere to give the mentioned benzoyl derivatives.

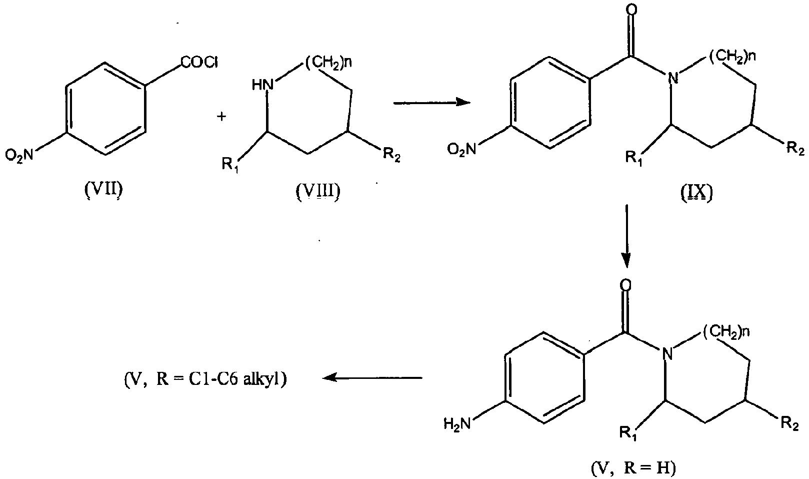

As already said, the compounds of the invention exert their activity in virtue of their ability to specifically inhibit both enzymes pteridine reductase 1 and dihydrofolate reductase at the same time.

According to the invention, the compounds of formula (Ia) can inhibit the two enzymes independently or can inhibit PTR1 in combination with known antifolates; the enzyme profile has been obtained against a number of different enzymes as reported below. The compounds of the invention were tested against the following enzyme library: TcPTR1, LmPTRI , LmDHFR, LcTS, EcTS, CnTS, hTS, DHFR. The proteins were all purified following the known procedures (ref). The enzymes were purified through cromathographic methods that usually require a prepurification step using a ion exchange resin, than the eluted active fractions were pooled and a second purification step follows were a hydrophobic resin and sometimes a gel filtration resin is used as the final step. The protein is characterized using Mass Spectrometry and Specific Activity and concentration is detected. Moreover Km for the substrates is useful to set up the inhibition experiments. In general the protein is stored at -80°C and defrozen in small quantities when necessary. The biological assays are usually performed following the standard TS assay, the standard DHFR assay; the inhibition of the PTR1 enzyme has been performed using dihydrofolic acid as substrate and NADPH as cofactor.

Preferred compounds of this invention are compounds of formula Ia included in the following table. The compounds included in the following table which do not fall within the scope of formula (Ia) of claim 1 are clearly reference examples.

| **Structure** | **TS** | | **DHFR** | | **PTR1** | |
|---|---|---|---|---|---|---|
| | ***h* KᵢµM** | ***Lm* KᵢµM** | ***h* KᵢµM _{P}M** | ***Lm* KᵢµM** | ***Lm* KᵢµM** | ***Tc* KᵢµM** |
| 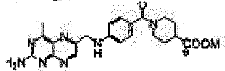 | NI | NI | 10 | 4 | 0.1 | 7 |
| 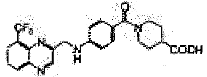 | 41 | NI | 56 | 40 | 7 | 75 |
| 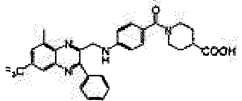 | 13 | NI | 54 | 17 | - | 20 |
| 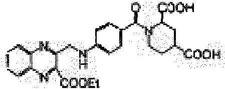 | 15 | NI | 24 | 75 | NI | 38 |
| 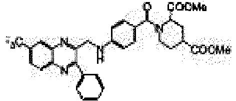 | 13 | NI | NI | 88 | NI | 40 |
| 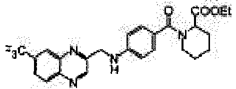 | 12 | 86 | NI | 27 | 6 | 106 |
| 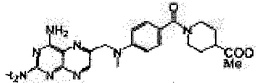 | NI | NI | 0.8 | - | 0.037 | - |
| 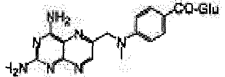 | - | 0.6 | 3.4^{#} | 130^{##} | 0.18* | 0.11 |
| 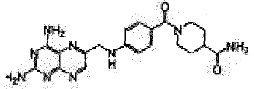 | NI° | | 15.4 | | 0.39 | |
| 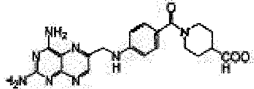 | NI° | | 0.59 | | 4.17 | |
| 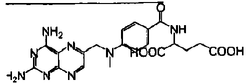 | 0.6 | | | 0.04 | | 0.18 |
| 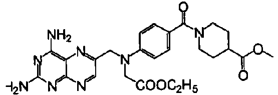 | NI° | | | NI° | | 0.10 |
| 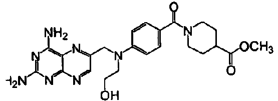 | 64.5 | | | 4.330 | | 0.030 |

In the Table NI means no inhibition at 190µM; - is a symbol that means not tested; * this symbol means that the compounds have been tested at the maximum concentration of 75µM; # this symbol means that Ki (inhibition constant) is expressed in pM; ## this symbol means that Ki (inhibition constant) is expressed in pM.

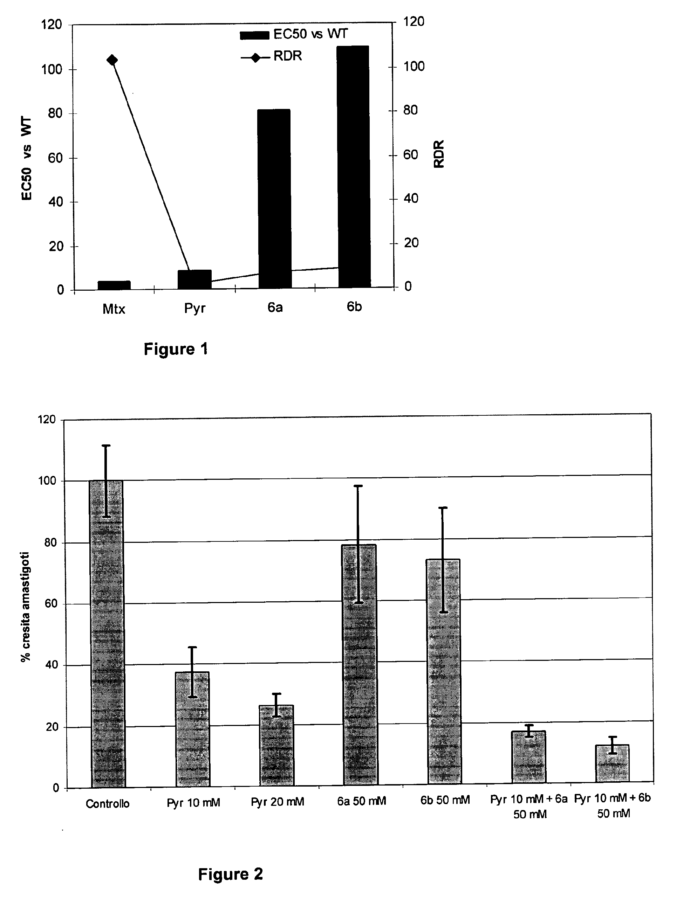

A further aspect of the present invention relates to the capacity of the compounds of formula (Ia) to inhibit the parasite growth as single agents or in combinations with antiparasitic agents, preferably antifolate compounds, most preferably inhibitors of parasitic Dihydrofolate reductase such as pyrimetamine. Compounds of formula (Ia) are able to inhibit the parasite growth with EC50, Effective Concentration leading to 50% of maximum effect, ranging from 40% to 100% (Figure 1). A further aspect of the present invention relates to the capacity to inhibit parasite growth, showing RDR, i.e. the relative index given by the ratio between the EC50 obtained from the PTR1 overexpressing lines by the EC50 value obtained from WT lines, that varies between 1 and 20, Figure 1. Additionally, the activity of selected compounds were tested against the amastigote intracellular forms preferably kinetoplastidae, preferably Trypanosoma cruzi in particular in association with some antiparasitic agents, preferably antifolates compounds, preferably compounds inhibitors of parasitic Dihydrofolate reductase. The results are reported in Figure 2. In the following tables the above described results are reported.

**Table 1 - EC50 vs WT parasites and RDR values (Figure 1)**

| | EC50 vs WT | Standard deviation | EC 50 vs PTR overexpressed | Standard deviation | RDR | Standard deviation |
|---|---|---|---|---|---|---|
| Mtx | 3,6 | 1,3 | 390,3 | 240,0 | 104,1 | 28,4 |
| Pyr | 8,7 | 4,5 | 18,4 | 3,2 | 2,3 | 0,8 |
| 6a | 81,1 | 59,3 | 428,7 | 66,1 | 7,6 | 6,4 |
| 6b | 109,3 | 64,6 | 871,4 | 29,1 | 9,6 | 5,4 |

**Table 2. Intracellular growth percentage of the Tripanosoma cruzi amastigote form after 72 h exposure to the different inhibitors (Figure 2).**

| % amastigotes growth | % amastigotes growth (medium value) | Error (Standard deviation) |
|---|---|---|
| Control | 100 | 11,6 |
| Pyr 10 M | 37,4 | 8,0 |
| Pyr 20 M | 26,3 | 3,7 |
| 6a 50 M | 78,3 | 19,0 |
| 6b 50 M | 73,0 | 17,0 |
| Pyr 10 M + 6a 50 M | 17,0 | 1,9 |
| Pyr 10 M + 6b 50 M | 12,1 | 2,8 |

In a third aspect, the present invention provides pharmaceutical compositions comprising the compounds of formula (Ia), as previously defined, for the preparation of a drug for the treatment, prevention or inhibition of an infective pathology caused and/or sustained by parasites, whose treatment can be done or favoured inhibiting the activity of Pteridine reductase and Dihydrofolate reductase of pathogen bacteria including but not limited to, Leishmania species and Trypanosoma species, in animals, preferably mammalian subjects, most preferably human subjects.

In a further aspect, the present invention provides pharmaceutical compositions comprising the compounds of formula (Ia) for the preparation of a drug for the treatment, prevention or inhibition of an infective pathology caused and/or sustained by parasites, as previously described, and an acceptable excipient. Therefore the compound can be prepared for oral or parenteral administration for the treatment of parasitic infections caused to animal subjects, preferably mammal subjects, most preferably human subjects.

As a specific feature, a compound of the present invention is to be mixed with excipient conventional pharmaceutical products and can be used in the formulation of tablets, capsules, suspensions, syrups, and others. Such pharmaceutical substances are likely to contain 0.1 to 90 % of their active compound by weight, and generally from 10 to 30 %. Pharmaceutical compositions can contain ordinary carriers and excipients, likewise maize starch, lactose, sucrose, microcrystalline cellulose, caolin, mannitol, dicalcium phosphate, sodium chloride and alginic acid. Disintegrators usually used in the formulations of the present invention include microcrystalline cellulose, maize starch, and alginic acid glycolate of sodium starch.

A liquid composition may generally consist of a suspension or solution of the compound in a carrier or suitable liquid carrier, for example ethanol, glycerine, sorbitol, nonaqueous solvent as polyethylene glycol, oleum or water with suspensive agent, preservative, surfactant, wetting agent, flavouring or colouring agents.

Otherwise, any liquid formulation can be obtained using a reconstitutive powder. E.g. a powder containing the active compound, the suspensive agent, saccharose and a sweetener might be reconstituted with water, furthermore a syrup may be prepared using a specific powder which contains the active compound, saccharose and sweetener.

A composition in the form of tablet might be prepared by using any suitable carrier or any suitable carrier usually used for solid formulations. Examples of such carriers include magnesium stearate, starch, lactose, saccharose, microcrystalline cellulose and ligands, e.g. povidone. Where appropriate, the tablets may be prepared with coatings, coloured if wished, with enteric coatings or as to provide controlled release of active ingredients in the intestinal tract. Moreover, the active ingredient can be formulated as to provide controlled release of active ingredients as tablet included in the hydrophilic or hydrophobic matrix.

A composition in the form of capsule can be prepared using ordinary process of incapsulation, e.g including the active ingredient and excipients in one capsule of hard gelatine. Alternatively a semi-solid matrix of an active ingredient and polyethylene glycol of high molecular weight can be prepared and inserted in a capsule of hard gelly; or a solution of the active ingredient in polyethylene glycol or in an oleum suspension for food, e.g. liquid paraffin or fractioned oleum of coconut, can be prepared and inserted in a capsule of soft gelly.

Ligands for tablets that can be included are acacia, methylcellulose, sodium carboxymethylcellulose, povidone, hydroxypropylmethylcellulose, sucrose, starch and ethylcellulose. Lubrificant that can be used include magnesium stearate or other metallic stearate, stearic acid, fluid silicone, talcum, wax, oleum and colloidal silica. Flavouring agents as menta piperita, cherry flavour or similar, can also be used. Moreover it can be desirable adding a colouring agent to make more attractive the formulation or to make the product identification easier. The compounds of the invention can be considered active whenever they are administered through parenteral, intramuscular, intratecal or intravenous route.

On the other hand, a typical composition for intravenous or intratecal administration may consist of a sterile water and isotonic solution which may contain the active ingredient and dextrose, or a mixture of dextrose and sodium chloride. Optionally a co-solvent, as polyethylene glycol, chelating agent, likewise ethylendiamine tetraacetic acid and one antioxidant, e.g. sodium metabisulfite can be included in the formulation. Alternatively, the hereof solution might be dried by freezing/sublimation and then reconstituted through any suitable solvent just before its administration.

The compounds of the invention which result to be active in rectal route administration can be formulated as suppository. A typical suppository formulation may consist of the active ingredient connected with a ligand agent and/or lubrificant likewise gelatine or cacao butter or other wax or vegetable fat or low melting synthetic agents. The compounds of the invention which are active in topic administration can be formulated in the transdermal formulation or transdermal delivery system (dermal patches). These compositions include, for example, coating, reservoir of the active ingredient, bandage and contact adhesive. These dermal patches are likely to be used to provide a continuous or discontinuous infusion of the compounds described in this invention within limited quantities. Production and use of dermal patches for the release of pharmaceutical agents are well known in the art. See, e.g., US Patent No. 5,023,252 granted on the 11th June 1991. The active ingredient has its effects among a long range of dosages and it is generally administrated in an effective pharmaceutical amount. The hereof dosage which shall be administrated is supposed to be decided by the physician in charge, especially in consideration of relevant circumstances, as age, weight and any kind of patient's reaction to the drug, the severity of patient's symptoms and the like.

Proper dosages may vary between 0.01-100 mg/kg/day, preferably 0.1-50 mg/kg/day. For what concerns a 70 Kg average man the appropriate dosage is to be 0.7 mg to 7 g per day, or more preferably 7 mg to 3.5 g per day.

Any other formulation suitable for using the present invention may be found in Remington's Pharmaceutical Sciences, Mace Publishing Company, Philadelphia, PA 17th edition (1985).

The following examples illustrate but do not limit the invention.

### Example 1: general method for the preparation of compounds V.

Compounds V in particular where
R5 = H; R6 = COOMe; n = 2, R5 = COOEt; R6 = H; n = 1 , R5 = COOEt; R6 = H; n = 2, R5 = COOMe; R6 = COOMe; n = 2

A mixture of 4-nitrobenzoyl derivatives (3.5 mmol) in ethanol (30 ml) and 10% palladised charcoal (100 mg) was hydrogenated at 3 atm and 15°C within 30 min.

The catalyst was filtered off through a filter paper, washed with ethanol and the mother liquors evaporated to dryness to give the corresponding amino benzoyl derivatives 4a-d (yields: 72-92%).

### Example 2: N-[4-(2,4-diaminopteridin-6-ylmethylamino)benzoyl]-piperidin-4-carboxy methyl ester.

To a solution of methyl 1-(4-aminobenzoyl)piperidine-4-carboxylate (100 mg, 0.36 mmol) in 3ml of DMA was added an equimolar amount of 6-bromomethyl-2,4-diaminopteridine hydrobromide synthesized as referenced. The solution was stirred and kept at room temperature for 90 h. The solvent was removed by evaporation in vacuo (<1 mm Hg, bath to 45°C). The residue was purified by flash chromatography using CHCl₃/MeOH (9:1 , vol/vol) as eluent to afford the desired compound as a solid (65 mg, 50% yield).

### Example 3: Methyl 1-{4-(2,4-diaminopteridin-6-ylmethyl) methylamino]benzoyl}4-carboxy methyl piperidine carboxylate.

To a solution of methyl 1-(4-methylaminobenzoyl) piperidine-4-carboxylate (120 mg, 0.43 mmol) in 3 ml of DMA was added an equimolar amount of 6-bromomethyl- 2,4-diaminopteridine hydrobromide synthesized as reported. The solution was stirred and kept at room temperature for 84 h. The solvent was removed by evaporation in vacuo (<1mmHg, bath to 45°C). The residue was purified by flash chromatography using CHCl₃/MeOH (9:1, vol/vol) as eluent to afford the desired compound as a solid (110 mg, 50% yield). Characterization as dihydrobromide
IR (nujol): 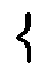 3300, 3110, 1728,1625. UV (EtOH): λmax 378, 276, 205. 1H-NMR (200-MHz, DMSO): δ 9.39 (s, 1H, exc. with D2O), 8.87 (s, 1H, exc. with D2O), 8.75 (br s, exc. with D2O, 2H), 8.67 (s,1H), 7.45 (br s, exc. with D2O, 2H), 7.27 (d, *J* = 7.6 Hz, 2H), 6.78 (d, *J* = 7.6 Hz, 2H), 4.87 (s, 2H), 4.10-3.90 (m, 2H), 3.65 (s, 3H), 3.25 (s, 3H), 3.10-2.95 (m, 2H), 2.70-2.55 (m, 1H), 1.93-1.80 (m, 2H), 1.70-1.55 (m, 2H). Mp = 203-205°C. GC/MS: m/z 612 [M]+. Anal. Calcd for C22H26F3N803
2HBr: C, 43.15; H, 4.61 ; N, 18.30. Found: C, 42.90; H, 4.68; N, 18.61.

### Example 4: 1-(4-(((2,4-diaminopteridin-6-yl)methyl)(2-hydroxyethyl)amino) benzoyl)piperidine-4-carboxylate.

A mixture of the 6-(bromomethyl)pteridine-2,4-diamine hydrobromide (0.3 mmol), prepared according to the literature procedure1 by treatment of (2,4-diaminopteridin-6-yl)methanol hydrochloride (Aldrich) with dibromotriphenylphosphorane (Ph₃PBr₂) in Me₂NAc, and an excess of the amines (0.6 mmol), synthesized as reported later, in Me₂NAc (5ml) was stirred at room temperature until the reaction was complete. The solvent was removed under reduced pressure, and the residue was purified by flash column chromatography using a mixture of chloroform/methanol as eluent in different ratios. These compounds are prepared with a yield of 42% to give a solid white powder with the following properties: Mp > 300°C; ¹H NMR (CDCl₃/DMSO-*d₆*): δ 8.71 (1H, s, pteridin-H), 7.65 (2H, br s, exc. with D₂O, NH₂), 7.20 (2H, d, J = 8.2 Hz, aryl-H), 6.73 (2H, d, J = 8.8 Hz, aryl-H), 6.64 (1 H, br s, exc. with D₂O, NH), 6.34 (2H, s, exc. with D₂O, NH₂) , 4.49 (2H, d, CH₂), 4.30-4.08 (2H, m, piperidin-H), 3.02-2.90 (2H, m, piperidin-H), 2.60-2.30 (1H, m, piperidin-H), 1.85-1.50 (4H, m, piperidin-H); IR (nujol): ν 3401 , 3181 , 1645, 1611 cm⁻¹; UV (EtOH): λₘₐₓ 368, 264, 205 nm; LC/MS: m/z 422 [M + 1]. Anal. Calcd. For C₂₀H₂₃N₉O₂: C, 57.00; H, 5.50; N, 29.91 ; found C, 56.88; H, 5.44; N, 29.79.

### Example 5 (reference example): Methyl 1-(4-(((2,4-diaminopteridin-6-yl) methyl)(2-ethoxy-2-oxoethyl) amino) benzoyl) piperidine-4-carboxylate.

A mixture of the 6-(bromomethyl)pteridine-2,4-diamine hydrobromide (0.3 mmol), prepared according to the literature procedure¹ by treatment of (2,4-diaminoptehdin-6-yl)methanol hydrochloride (Aldrich) with dibromotriphenylphosphorane (Ph₃PBr₂) in Me₂NAc, and an excess of the amines (0.6 mmol), synthesized as reported later, in Me₂NAc (5ml) was stirred at room temperature until the reaction was complete. The solvent was removed under reduced pressure, and the residue was purified by flash column chromatography using a mixture of chloroform/methanol as eluent in different ratios. These compounds are prepared with a yield of 42% to give a solid white powder.

### Example 6 (reference example): 1-(4-((2,4-diaminopteridin-6-yl)methylamino)benzoyl) piperidine-4- carboxamide.

This compound was prepared in 42% yield by the protocol described in the general procedure starting from 1 and 1-(4-aminobenzoyl)piperidine-4-carboxamide (2a) for 7 days; the compound was purified by flash choromatography (chloroform:methanol = 8:2, Rf 0.28) to give a white solid; mp >300°C.
¹H NMR (CDCl₃/DMSO-*d₆*): δ 8.71 (1H, s, pteridin-H), 7.65 (2H, br s, exc. with D₂O, NH₂), 7.20 (2H, d, J = 8.2 Hz, aryl-H), 6.73 (2H, d, J = 8.8 Hz, aryl-H), 6.64 (1H, br s, exc. with D₂O, NH), 6.34 (2H, s, exc. with D₂O, NH₂), 4.49 (2H, d, CH₂), 4.30-4.08 (2H, m, piperidin-H), 3.02-2.90 (2H, m, piperidin-H), 2.60-2.30 (1H, m, piperidin-H), 1.85-1.50 (4H, m, piperidin-H); IR (nujol): ν 3401 , 3181 , 1645, 1611 cm⁻¹; UV (EtOH) : λₘₐₓ 368, 264, 205 nm; LC/MS: m/z 422 [M + 1]. Anal. Calcd. For C₂₀H₂₃N₉O₂: C, 57.00; H, 5.50; N, 29.91 ; found C, 56.88; H, 5.44; N, 29.79.

### Example 7: General method for the preparation of compounds with formula Ib which are not part of the invention:

To a solution of 4-amino benzoyl derivatives of formula V (0.57 mmol) in propanol or ethanol was added an equimolar amount of 2-chloroquinoxaline of formula VI prepared according to general procedures as reported in literature. The reaction mixture was stirred under reflux for a variable time and, eventually, was allowed to cool to room temperature. The yellow precipitates of non carboxilated compounds were filtered off and washed with 1- propanol.
For the other compounds the solvent was evaporated under reduced pressure and the residue was purified by flash chromatography over silica gel using a mixture of petrol ether (40-60°C)/ethyl acetate as eluent in different ratios to afford the corresponding quinoxalines (yields: 17-64%). Methods for the preparation of compounds with formula Ib. To a solution of the derivatives 9b, d, f-s (0.12 mmol) in a mixture of MeOH (2 ml) and THF (8 ml) was added LiOH (0.21 mmol). The reaction mixture was stirred for a variable time at room temperature and, eventually, it was diluted with water. Then the organic solvent was evaporated in vacuo and the residue was made neutral with an equimolar amount of 1 M HCl in aqueous solution.

The compounds 10b, d, f-s were filtered or extracted with CHCl3. The products were purified by flash chromatography using a mixture of CHCl3/MeOH (9:1, vol/vol) as eluent to afford the carboxylic acid 10b, d, f-s (yields: 47-79%).

## Claims

1. A compound of formula (Ia): 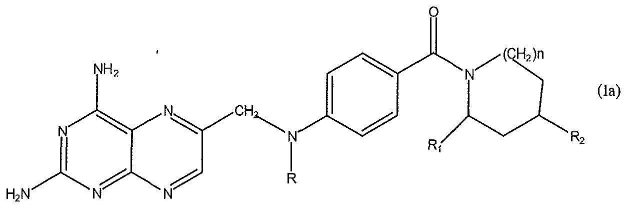 wherein:
R is hydrogen or CH₃, CH₂CH₂OH, COOH, COOCH₃, COOC₂H₅;
R₁ is hydrogen or COOH; R₂ is hydrogen, COOH or COOCH₃; and
n is 0 or 1.

2. The compound according to claim 1, which is:
1-[4-(2,4-diaminopteridin-6-ylmethylamino)benzoyl] piperidin-2-carboxylic acid;
1-[4-(2,4-diaminopteridin-6-ylmethylamino)benzoyl] piperidin-4-carboxylic acid;
1-[4-(2,4-diaminopteridin-6-ylmethylamino)benzoyl] piperidin-2,4-dicarboxylic acid;
1-[4-(2,4-diaminopteridin-6-ylmethylamino)benzoyl] pyrrolidin-2-carboxylic acid;
1-[4-(2,4-diaminopteridin-6-ylmethylamino)benzoyl] pyrrolidin-4-carboxylic acid;
1-[4-(2,4-diaminopteridin-6-ylmethylamino)benzoyl] pyrrolidin-2,4-dicarboxylic acid;
1-{4-[(2,4-diaminopteridin-6-ylmethyl)(methyl)amino] benzoyl}pyrrolidin-2-carboxylic acid;
1-{4-[(2,4-diaminopteridin-6-ylmethyl) (methyl)amino]benzoyl}pyrrolidin-4-carboxylic acid;
1-{4-[(2,4-diaminopteridin-6-ylmethyl) (methyl)amino]benzoyl}pyrrolidin-2, 4-dicarboxylic acid;
1-{4-[(2,4-diaminopteridin-6-ylmethyl)(methyl)amino]benzoyl}piperidin-2-carboxylic acid;
1-{4-[(2,4-diaminopteridin-6-ylmethyl)(methyl)amino]benzoyl}piperidin-4-carboxylic acid;
1-{4-[(2,4-diaminopteridin-6-ylmethyl)(methyl)amino]benzoyl}piperidin-2, 4-dicarboxylic acid
1-[4-(2,4-diaminopteridin-6-ylmethylamino)benzoyl]-piperidin-4-carboxyl-methyl ester.

3. The compound according to claim 1, wherein the formula is:

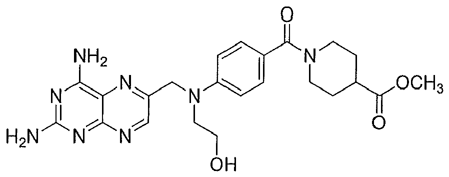

4. The compound according to any claims 1 to 3 for use as a medicament.

5. The compound according to claim 4 for use in the treatment of an infective pathology caused and/or sustained by parasites.

6. The compound according to claim 4 or 5, for use in the treatment of protozoarian infections.

7. The compound according to any one of claims 4 to 6, for use in the treatment of Leihsmaniasis or Tripanosomiasis.

8. The compound according to any one of claims 4 to 7, for use in association with an inhibitor of dihydrofolate reductase.

9. The compound for use according to claim 8, in which said inhibitor of dihydrofolate reductase is pyrimetamine.

## Patentansprüche

1. Verbindung der Formel (Ia): 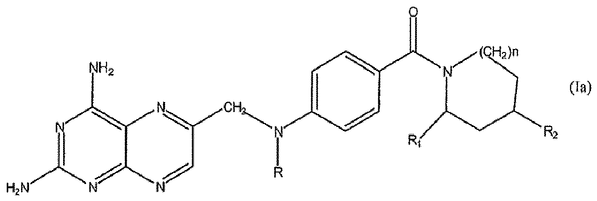 wobei
R Wasserstoff oder CH₃, CH₂CH₂OH, COOH, COOCH₃, COOC₂H₅ ist, R₁ Wasserstoff oder COOH ist, R₂ Wasserstoff, COOH oder COOCH₃ ist
und
n 0 oder 1 ist.

2. Verbindung nach Anspruch 1, die besteht aus:
1-[4-(2,4-Diaminopteridin-6-Ylmethylamino)Benzoyl]-Piperidin-2-Carboxylsäure;
1-[4-(2,4-Diaminopteridin-6-Ylmethylamino)Benzoyl]-Piperidin-4-Carboxylsäure;
1-[4-(2,4-Diaminopteridin-6-Ylmethylamino)Benzoyl]-Piperidin-2,4-Dicarboxylsäure;
1-[4-(2,4-Diaminopteridin-6-Ylmethylamino)Benzoyl]-Pyrrolidin-2-Carboxylsäure;
1-[4-(2,4-Diaminopteridin-6-Ylmethylamino)Benzoyl]-Pyrrolidin-4-Carboxylsäure;
1-[4-(2,4-Diaminopteridin-6-Ylmethylamino)Benzoyl]-Pyrrolidin-2,4-Dicarboxylsäure;
1-{4-[(2,4-Diaminopteridin-6-Ylmethyl)(Methyl)Amin]Benzoyl}-Pyrrolidin-2-Carboxylsäure;
1-{4-[(2,4-Diaminopteridin-6-Ylmethyl)(Methyl)Amin]Benzoyl}-Pyrrolidin-4-Carboxylsäure;
1-{4-[(2,4-Diaminopteridin-6-Ylmethyl)(Methyl)Amin]Benzoyl}-Pyrrolidin-2,4-Dicarboxylsäure;
1-{4-[(2,4-Diaminopteridin-6-Ylmethyl)(Methyl)Amin]Benzoyl}-Piperidin-2-Carboxylsäure;
1-{4-[(2,4-Diaminopteridin-6-Ylmethyl)(Methyl)Amin]Benzoyl}-Piperidin-4-Carboxylsäure;
1-{4-[(2,4-Diaminopteridin-6-Ylmethyl)(Methyl)Amin]Benzoyl}-Piperidin-2,4-Dicarboxylsäure;
1-[4-(2,4-Diaminopteridin-6-Ylmethylamin)Benzoyl]-Piperidin-4-Carboxyl-Methylester.

3. Verbindung nach Anspruch 1, wobei die Formel ist:

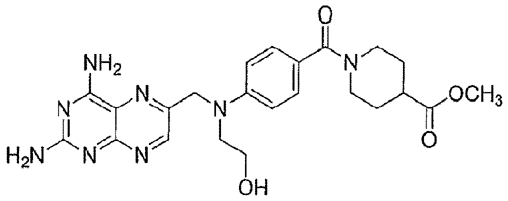

4. Verbindung nach einem der Ansprüche 1 bis 3 für den Gebrauch als Medikament.

5. Verbindung nach Anspruch 4 für den Gebrauch bei der Behandlung einer Infektionskrankheit, die von Parasiten hervorgerufen und/oder gefördert wird.

6. Verbindung nach Anspruch 4 oder 5 für den Gebrauch bei der Behandlung von Protozoeninfektionen.

7. Verbindung nach einem der Ansprüche 4 bis 6 für den Gebrauch bei der Behandlung von Leihsmaniose oder Trypanosomiasis.

8. Verbindung nach einem der Ansprüche 4 bis 7 für den Gebrauch in Assoziation mit einem Inhibitor der Dihydrofolatreduktase.

9. Verbindung für den Gebrauch nach Anspruch 8, wobei der Inhibitor der Dihydrofolatreduktase Pyrimethamin ist.

## Revendications

1. Composé de la formule (Ia) : 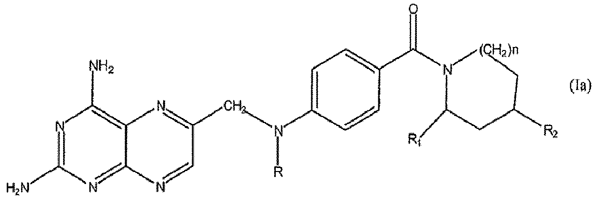 dans lequel :
R est hydrogène ou CH₃, CH₂CH₂OH, COOH, COOCH₃, COOC₂H₅ ;
R₁ est hydrogène ou COOH ; R₂ est hydrogène, COOH ou COOCH₃ ;
et
n est 0 ou 1.

2. Composé selon la revendication 1, qui est :
acide 1-[4-(2,4-diaminoptéridine-6-yl-méthylamino)benzoyl] pipéridine-2-carboxylique ;
acide 1-[4-(2,4-diaminoptéridine-6-yl-méthylamino)benzoyl] pipéridine-4-carboxylique ;
acide 1-[4-(2,4-diaminoptéridine-6-yl-méthylamino)benzoyl] pipéridine-2,4-dicarboxylique ;
acide 1-[4-(2,4-diaminoptéridine-6-yl-méthylamino)benzoyl] pyrrolidine-2-carboxylique ;
acide 1-[4-(2,4-diaminoptéridine-6-yl-méthylamino)benzoyl] pyrrolidine-4-carboxylique ;
acide 1-[4-(2,4-diaminoptéridine-6-ylméthylamino)benzoyl] pyrrolidine-2,4-dicarboxylique ;
acide 1-{4-[(2,4-diaminoptéridine-6-yl-méthyl)(méthyl)amino]benzoyl}pyrrolidine-2-carboxylique ;
acide 1-{4-[(2,4-diaminoptéridine-6-yl-méthyl)(méthyl)amino]benzoyl}pyrrolidine-4-carboxylique ;
acide 1-{4-[(2,4-diaminoptéridine-6-yl-méthyl)(méthyl)amino]benzoyl}pyrrolidine-2,4-dicarboxylique ;
acide 1-{4-[(2,4-diaminoptéridine-6-yl-méthyl)(méthyl)amino]benzoyl}pipéridine-2-carboxylique ;
acide 1-{4-[(2,4-diaminoptéridine-6-yl-méthyl)(méthyl)amino]benzoyl}pipéridine-4-carboxylique ;
acide 1-{4-[(2,4-diaminoptéridine-6-yl-méthyl)(méthyl)amino]benzoyl}pipéridine-2,4-dicarboxylique ;
ester méthylique 1-[4-(2,4-diaminoptéridine-6-yl-méthylamino)benzoyl]-pipéridine-4-carboxylique.

3. Composé selon la revendication 1, dans lequel la formule est :

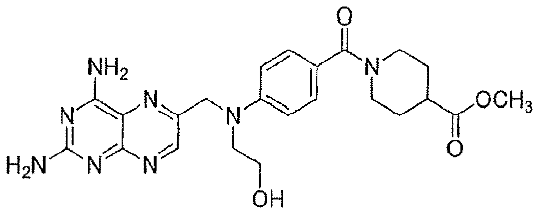

4. Composé selon l'une quelconque des revendications 1 à 3 pour son utilisation en tant que médicament.

5. Composé selon la revendication 4 pour son utilisation dans le traitement d'une pathologie infectieuse provoquée et/ou transmise par des parasites.

6. Composé selon les revendications 4 ou 5 pour son utilisation dans le traitement des infections à protozoaires.

7. Composé selon l'une quelconque des revendications 4 à 6 pour son utilisation dans le traitement de la leishmaniose ou de la trypanosomiase.

8. Composé selon l'une quelconque des revendications 4 à 7 pour son utilisation en association avec un inhibiteur de la dihydrofolate réductase.

9. Composé pour son utilisation selon la revendication 8, dans lequel ledit inhibiteur de la dihydrofolate réductase est pyriméthamine.
